# EUROPÄISCHE PATENTSCHRIFT

(11) **EP 1 024 779 B1**
(45) Veröffentlichungstag und Bekanntmachung des Hinweises auf die Patenterteilung: **26.02.2003**
(21) Anmeldenummer: 98952696.7
(22) Anmeldetag: 05.10.1998
(51) Int. Cl.: A61K 7/13

(54) **VERWENDUNG VON HALOGENIERTEN TRIPHENYLMETHANSULFONATEN ZUM FÄRBEN VON KERATINHALTIGEN FASERN**
APPLICATION OF HALOGENATED TRIPHENYLMETHANE SULFONATES FOR DYING FIBERS CONTAINING KERATIN
UTILISATION DE SULFONATES DE TRIPHENYLMETHANE HALOGENES POUR COLORER DES FIBRES A BASE DE KERATINE

(30) Priorität: 14.10.1997 DE 19745293
(43) Veröffentlichungstag der Anmeldung: 09.08.2000
(73) Patentinhaber: Henkel Kommanditgesellschaft auf Aktien, 40191 Düsseldorf (DE)
(72) Erfinder: MÖLLER, Hinrich, D-40789 Monheim (DE); MEINIGKE, Bernd, D-51381 Leverkusen (DE)
(86) Internationale Anmeldenummer: EP9806307
(87) Internationale Veröffentlichungsnummer: WO99018915

(56) Entgegenhaltungen:
- EP-A- 0 471 105
- DE-A- 2 262 940
- US-A- 4 960 585
- US-A- 5 474 578
- CHEMICAL ABSTRACTS, vol. 115, no. 6, 12. August 1991 Columbus, Ohio, US; abstract no. 51801, "DYEING SYNTHETIC OR NATURAL FIBERS WITH PH INDICATORS" XP002099496 & ES 2 017 556 A16. Februar 1991

## Beschreibung

Die Erfindung betrifft die Verwendung von halogenierten Triphenylmethansulfonaten in Kombination mit Oxidationshaarfarbstoffen zum Färben von keratinhaltigen Fasern, insbesondere menschlichen Haaren, ein Mittel zum Färben von keratinhaltigen Fasern, das die obengenannte Farbstoffkombination enthält, sowie ein Verfahren zum Färben von keratinhaltigen Fasern.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Die direktziehenden Farbstoffe bilden die Farbe unmittelbar auf der Faser aufgrund ihrer Eigenfarbe. Die Farbe auf der Faserverblaßt im Laufe der Zeit oder wird während der Wäsche ausgewaschen. Verglichen mit den Oxidationsfarbstoffen sind die Intensitität der Färbung und die Echtheitseigenschaften geringer.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken.

Direktziehende Farbstoffe werden unter schonenderen Bedingungen appliziert. Sie werden oft auch in Kombination mit Oxidationsfarbstoffen eingesetzt, um bestimmte Farbnuancen zu erhalten.

Aufgabe der vorliegenden Erfindung ist es, Färbemittel bereitzustellen, mit denen eine große Vielfalt von Farbnuancen erhalten werden kann. Eine Anfärbung der Hautpartien sollte möglichst vermieden werden. Darüber hinaus dürfen die Färbemittel kein oder lediglich ein sehr geringes Sensibilisierungspotential aufweisen.

Überraschenderweise wurde gefunden, daß bestimmte halogenierte Triphenylmethansulfonate hervorragend zum Färben von keratinhaltigen Fasern geeignet sind. Die Verbindungen können in organischen, wäßrigen oder organischwäßrigen Zubereitungen aufgebracht werden und ergeben Ausfärbungen mit hervorragender Brillanz und Farbtiefe und führen zu vielfältigen Farbnuancen.

Gegenstand der vorliegenden Erfindung ist die Verwendung von halogenierten Triphenylmethansulfonaten der folgenden Formel I 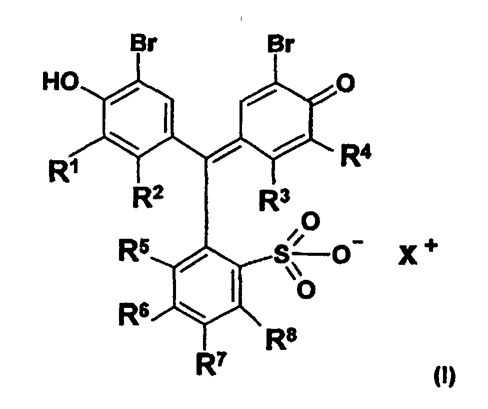 worin
R¹, R², R³ und R⁴ jeweils unabhängig voneinander für ein Wasserstoffatom, ein Chloratom, ein Bromatom oder eine Hydroxygruppe stehen,
R⁵, R⁶, R⁷ und R⁸ jeweils unabhängig voneinander für ein Wasserstoffatom, ein Chloratom oder ein Bromatom stehen und
X ein Wasserstoff-, Alkali- oder Erdalkaliatom oder eine Ammoniumgruppe bedeutet,
in Kombination mit Oxidationshaarfarbstoffen zum Färben von keratinhaltigen Fasern, insbesondere menschlichen Haaren.

Ein weiterer Gegenstand der vorliegenden Erfindung ist ein Mittel zum Färben von keratinhaltigen Fasern, insbesondere menschlichen Haaren, das halogenierte Triphenylmethansulfonate mit der folgenden Formel I 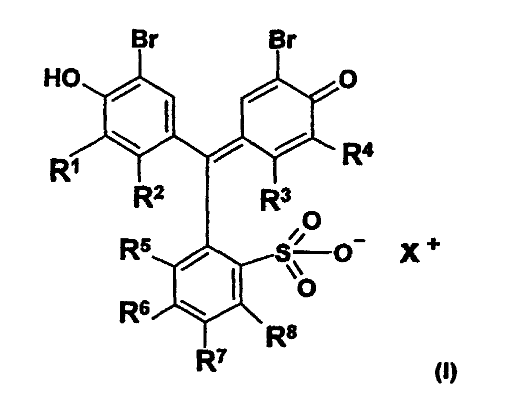 worin
R¹, R², R³ und R⁴ jeweils unabhängig voneinander für ein Wasserstoffatom, ein Chloratom, ein Bromatom oder eine Hydroxygruppe stehen,
R⁵, R⁶, R⁷ und R⁸ jeweils unabhängig voneinander für ein Wasserstoffatom, ein Chloratom oder ein Bromatom stehen und
X ein Wasserstoff-, Alkali- oder Erdalkaliatom oder eine Ammoniumgruppe bedeutet, in Kombination mit Oxidationshaarfarbstoffen enthält.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern verwendet werden.

Geeignete Verbindungen mit der Formel I sind Brompyrogallolrot, Bromphenolblau, Tetraphenolblau, Bromphenolrot, Bromthymolblau, Bromkresolgrün und Bromkresolpurpur.

Die Verbindungen mit der Formel I sind im erfindungsgemäßen Färbemittel vorzugsweise in einer Menge von 0,03 bis 65, insbesondere 1 bis 40 mmol, bezogen auf 100 g des gesamten Färbemittels enthalten.

Die Nuancenvielfalt und die Farbechtheit der erfindungsgemäß eingesetzten Verbindungen mit der Formel I können durch Kombination dieser Verbindungen mit anderen direktziehenden Farbstoffen erweitert werden.

In allen Färbemitteln können auch mehrere verschiedene färbende Substanzen gemeinsam zum Einsatz kommen; ebenso können auch mehrere verschiedene Komponenten aus den Gruppen von Verbindungen mit primärer oder sekundärer Aminogruppe, von stickstoffhaltigen Heterocyclen, aromatischen Hydroxyverbindungen oder Aminosäuren gemeinsam verwendet werden, wie sie beispielsweise in der deutschen Patentanmeldung 197 17 224.5 beschrieben werden.

Die erfindungsgemäßen Färbemittel können eine breite Palette von Farbnuancen ergeben, die im Bereich von gelb, rot, blau, blaugrün und grün liegen; die Echtheitseigenschaften sind hervorragend, die Sensibilisierungspotentiale sehr gering.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z.B. aus der Gruppe der Nitrophenylendiamine, Nitroaminophenole, Anthrachinone, Indophenole oder der als Arianore bekannten Verbindungen, wie z.B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 sowie Pikraminsäure, 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid, 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Färbemittel auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Bezüglich der einzelnen verwendbaren Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch.Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Es ist nicht erforderlich, daß die fakultativ enthaltenen weiteren direktziehenden Farbstoffe oder die Oxidationsfarbstoffvorprodukte jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes,

Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid undloder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller. Dow Coming; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Alkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchiorid, Magnesiumsulfat, Ammoniumcarbonat, - Chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Zum Färben der keratinhaltigen Fasern, insbesondere zum Färben von menschlichen Haaren, werden die Färbemittel in der Regel in Form des wasserhaltigen, kosmetischen Trägers in einer Menge von 100 g auf das Haar aufgebracht, ca. 30 Minuten dort belassen und anschließend ausgespült oder mit einem handelsüblichen Haarshampoo ausgewaschen.

### Beispiele

### Herstellung einer Färbecreme

Zur Herstellung einer Färbecreme wurden 1,0 g Cetarylalkohol, 1,0 g Kokosalkohol, 1,1 g Natrium-Laureth-5-carboxylat, 0,05 g Propylparaben und 0,15 g Methylparaben bei 80°C aufgeschmolzen, mit 70 g 80°C heißem Wasser vermischt und unter starkem Rühren emulgiert. Anschließend ließ man die Emulsion unter schwachem Rühren abkühlen. Das Färbemittel wurde in 10 g 50°C heißem Wasser unter Zugabe von 3,0 g Ammoniumsulfat und Ammoniak gelöst. Die Farbstofflösung wurde zur Emulsion gegeben, mit Ammoniak auf pH 9 eingestellt und mit Wasser auf 100 g aufgefüllt. Anschließend wurde die Creme unter Rühren auf Raumtemperatur abgekühlt.

Die erhaltene Färbecreme wurde bei 30°C 30 Minuten lang auf eine blonde Haarsträhne aufgebracht. Die gefärbte Strähne wurde anschließend 30 Sek. mit lauwarmem Wasser gespült, im warmen (30-40°C) Luftstrom getrocknet und anschließend ausgekämmt. Danach wurden die Ausfärbungen visuell bei Tageslicht beurteilt.

Die jeweiligen Farbnuancen und Farbtiefen sind in der nachfolgenden Tabellen wiedergegeben.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

**Tabelle 1**

| Färbemittel | Konzentration c [Gew.-%] | Farbe | Intensität |
|---|---|---|---|
| 3,3'-Tetrabrom-m-kresolsulfonphthalein (Bromkresolgrün) | 1 | grautürkis | ++(+) |
| 5,5'-Dibrom-o-kresolsulfonphthalein (Bromkresolpurpur) | 1 | mattgrün | ++ |
| Brompyrogallolrot | 0,75 | glockenblumenviolett | ++ |
| Tetrabromphenolblau | 0,18 | blaugrün | ++(+) |
| Bromphenolblau | 0,5 | graublau | +++ |
| Bromthymolblau, Natriumsalz | 0,5 | gelbbraun | + |

## Patentansprüche

1. Verwendung von halogenierten Triphenylmethansulfonaten der folgenden Formel I 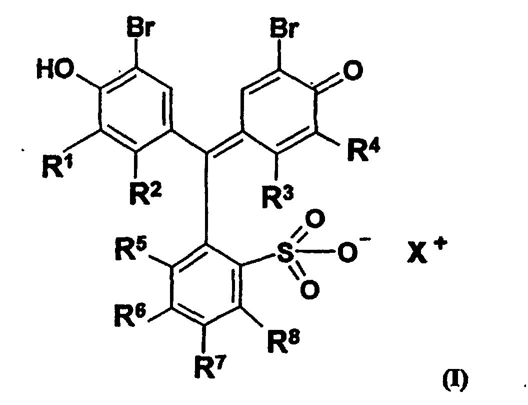 worin R¹, R², R³ und R⁴ jeweils unabhängig voneinander für ein Wasserstoffatom, ein Chloratom, ein Bromatom oder eine Hydroxygruppe stehen,
R⁵, R⁶, R⁷ und R⁸ jeweils unabhängig voneinander für ein Wasserstoffatom, ein Chloratom oder ein Bromatom stehen und
X ein Wasserstoff-, Alkali- oder Erdalkaliatom oder eine Ammoniumgruppe bedeutet,
in Kombination mit Oxidationshaarfarbstoffen zum Färben von keratinhaltigen Fasern, insbesondere von menschlichen Haaren.

2. Mittel zum Färben von keratinhaltigen Fasern, insbesondere menschlichen Haaren, das als eine färbende Komponente halogenierte Triphenylmethansulfonate mit der folgenden Formel I 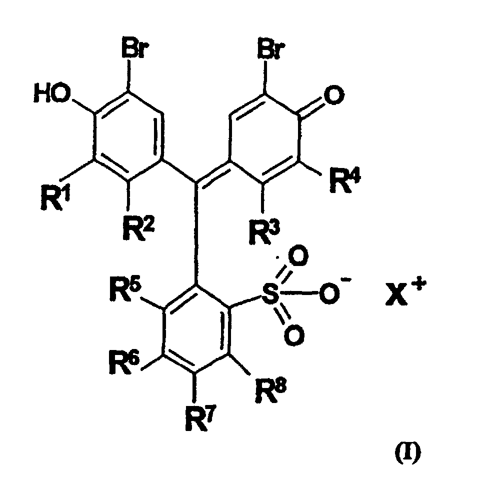 worin R¹, R², R³ und R⁴ jeweils unabhängig voneinander für ein Wasserstoffatom, ein Chloratom, ein Bromatom oder eine Hydroxygruppe stehen,
R⁵, R⁶, R⁷ und R⁸ jeweils unabhängig voneinander für ein Wasserstoffatom, ein Chloratom oder ein Bromatom stehen und
X ein Wasserstoff-, Alkali- oder Erdalkaliatom oder eine Ammoniumgruppe bedeutet,
in Kombination mit Oxidationshaarfarbstoffen enthält.

3. Mittel nach Anspruch 2, **dadurch gekennzeichnet, daß** die Verbindungen mit der Formel I ausgewählt sind aus Brompyrogallolrot, Bromphenolblau, Tetraphenolblau, Bromphenolrot und Bromthymolblau.

4. Mittel nach Anspruch 2 oder 3, **dadurch gekennzeichnet, daß** die Verbindungen mit der Formel I in einer Menge von jeweils 0,03 bis 65, insbesondere 1 bis 40 mmol, jeweils bezogen auf 100 g des gesamten Färbemittels, enthalten sind.

5. Mittel nach einem der Ansprüche 2 bis 4, **dadurch gekennzeichnet, daß** anionische, zwitterionische und/oder nichtionische Tenside eingesetzt werden.

6. Verfahren zum Färben von keratinhaltigen Fasern, worin ein Färbemittel, enthaltend halogenierte Triphenylmethansulfonate der folgenden Formel I 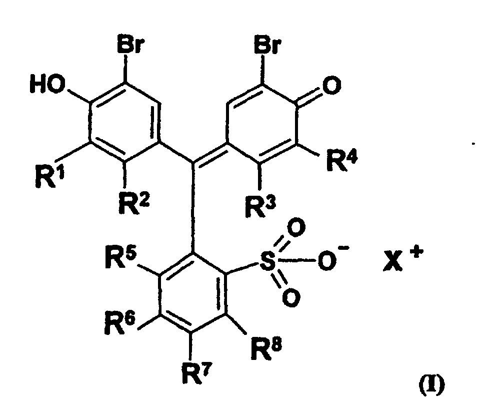 worin R¹, R², R³ und R⁴ jeweils unabhängig voneinander für ein Wasserstoffatom, ein Chloratom, ein Bromatom oder eine Hydroxygruppe stehen,
R⁵, R⁶, R⁷ und R⁸ jeweils unabhängig voneinander für ein Wasserstoffatom, ein Chloratom oder ein Bromatom stehen und
X ein Wasserstoff-, Alkali- oder Erdalkaliatom oder eine Ammoniumgruppe bedeutet,
in Kombination mit Oxidationshaarfarbstoffen sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. The use of halogenated triphenylmethane sulfonates corresponding to formula I: 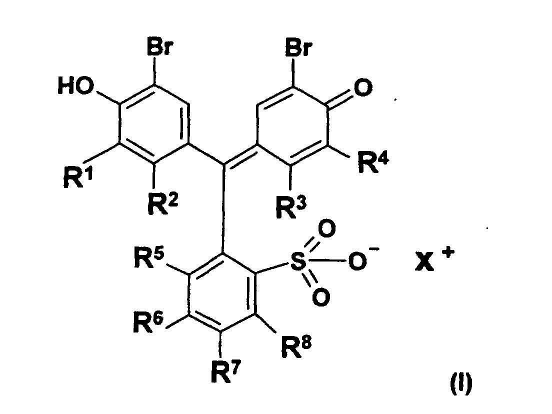 in which
R¹, R², R³ and R⁴ independently of one another represent a hydrogen atom, a chlorine atom, a bromine atom or a hydroxyl group,
R⁵, R⁶, R⁷ and R⁸ independently of one another represent a hydrogen atom, a chlorine atom or a bromine atom and
X is a hydrogen, alkali metal or alkaline earth atom or an ammonium group, in combination with oxidation hair dyes for coloring keratin-containing fibers, more particularly human hair.

2. A composition for coloring keratin-containing fibers, more particularly human hair, containing as a coloring component halogenated triphenylmethane sulfonates corresponding to formula I: 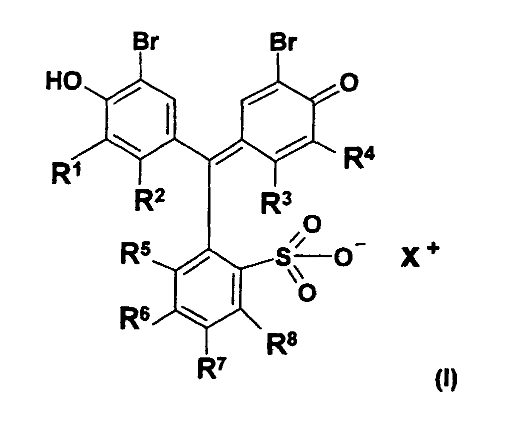 in which
R¹, R², R³ and R⁴ independently of one another represent a hydrogen atom, a chlorine atom, a bromine atom or a hydroxyl group,
R⁵, R⁶, R⁷ and R⁸ independently of one another represent a hydrogen atom, a chlorine atom or a bromine atom and
X is a hydrogen, alkali metal or alkaline earth atom or an ammonium group, in combination with oxidation hair dyes.

3. A composition as claimed in claim 2, **characterized in that** the compounds corresponding to formula I are selected from bromopyrogallol red, bromophenol blue, tetraphenol blue, bromophenol red and bromothymol blue.

4. A composition as claimed in claim 2 or 3, **characterized in that** the compounds of formula I are present in a quantity of 0.03 to 65 mmol and more particularly 1 to 40 mmol, based on 100 g of the colorant as a whole.

5. A composition as claimed in any of claims 2 to 4, **characterized in that** anionic, zwitterionic and/or nonionic surfactants are used.

6. A process for coloring keratin-containing fibers in which a colorant containing halogenated triphenylmethane sulfonates corresponding to formula I: 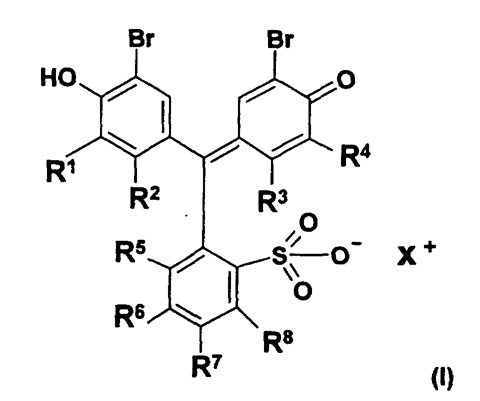 in which
R¹, R², R³ and R⁴ independently of one another represent a hydrogen atom, a chlorine atom, a bromine atom or a hydroxyl group,
R⁵, R⁶, R⁷ and R⁸ independently of one another represent a hydrogen atom, a chlorine atom or a bromine atom and
X is a hydrogen, alkali metal or alkaline earth atom or an ammonium group, in combination with oxidation hair dyes and typical cosmetic ingredients is applied to the keratin-containing fibers, left thereon for a time, usually about 30 minutes, and then rinsed out again or washed out with a shampoo.

## Revendications

1. Utilisation de sulfonates de triphénylméthane halogénés de la formule I ci-après 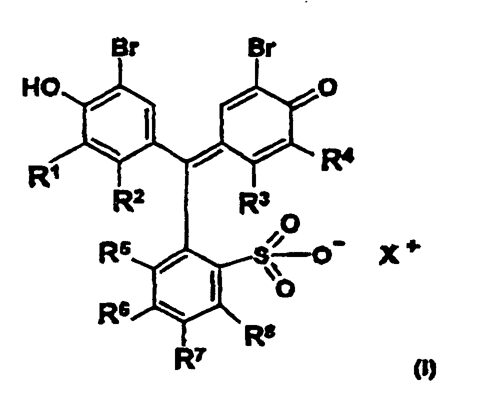 dans laquelle
R¹, R², R³ et R⁴ représentent chacun indépendamment des autres un atome d'hydrogène, un atome de chlore, un atome de brome ou un groupe hydroxyle,
R⁵, R⁶, R⁷ et R⁸ correspondent chacun indépendamment des autres à un atome d'hydrogène, un atome de chlore ou un atome de brome et
X est un atome d'hydrogène, un atome de métal alcalin ou alcalino-terreux ou un groupe ammonium,
en association avec des colorants d'oxydation pour cheveux ou pols pour colorer des fibres à base de kératine, en particulier les cheveux humains.

2. Produit pour colorer les fibres à base de kératine, en particulier les cheveux humains, qui contient comme composant colorant, des sulfonates de triphénylméthane halogénés de la formule I ci-après 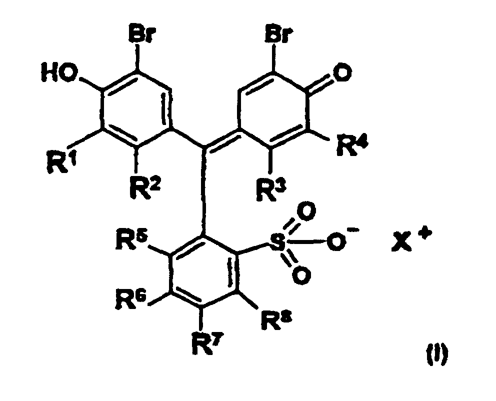 dans laquelle
R¹, R², R³ et R⁴ représentent chacun indépendamment des autres un atome d'hydrogène, un atome de chlore, un atome de brome ou un groupe hydroxyle,
R⁵, R⁶, R⁷ et R⁸ correspondent chacun indépendamment des autres à un atome d'hydrogène, un atome de chlore ou un atome de brome et
X est un atome d'hydrogène, un atome de métal alcalin ou alcalino-terreux ou un groupe ammonium,
en association avec des colorants d'oxydation pour cheveux ou pols.

3. Produit selon la revendication 2, **caractérisé en ce que** les composés de formule I sont sélectionnés parmi le rouge de bromopyrogallol, le bleu de bromophénol, le bleu de tétraphénol, le rouge de bromophénol et le bleu de bromothymol.

4. Produit selon la revendication 2 ou 3, **caractérisé en ce que** les composés de formule I sont contenus dans chaque cas dans une proportion de 0,03 à 65, en particulier de 1 à 40 mmol, dans chaque cas pour 100 g de la totalité du produit colorant.

5. Produit selon une des revendications 2 à 4, **caractérisé en ce que** des tensioactifs anioniques, zwitterioniques et/ou non ioniques sont utilisés.

6. Procédé pour colorer les fibres à base de kératine, dans lequel un produit colorant contenant des sulfonates de triphénylméthane halogénés de la formule I ci-après 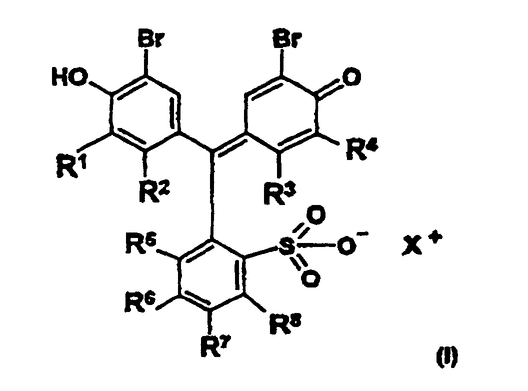 dans laquelle
R¹, R², R³ et R⁴ représentent chacun indépendamment des autres un atome d'hydrogène, un atome de chlore, un atome de brome ou un groupe hydroxyle,
R⁵, R⁶, R⁷ et R⁸ correspondent chacun indépendamment des autres à un atome d'hydrogène, un atome de chlore ou un atome de brome et
X est un atome d'hydrogène, un atome de métal alcalin ou alcalino-terreux ou un groupe ammonium,
est appliqué sur les fibres à base de kératine, en association avec des colorants d'oxydation pour cheveux ou pols et des ingrédients cosmétiques usuels, laissé sur la fibre pendant un certain temps, d'ordinaire environ 30 minutes, puis éliminé par rinçage ou par lavage avec un shampooing.
